# EUROPÄISCHE PATENTSCHRIFT

(11) **EP 1 697 523 B1**
(45) Veröffentlichungstag und Bekanntmachung des Hinweises auf die Patenterteilung: **03.10.2007**
(21) Anmeldenummer: 04804229.5
(22) Anmeldetag: 22.12.2004
(51) Int. Cl.: C12N 15/70

(54) **EXPRESSIONSVEKTOR ZUR ANTIBIOTIKAFREIEN EXPRESSION**
Expression vector and its use for an antibiotic free expression
VECTEUR D'EXPRESSION ET SON UTILISATION POUR UNE EXPRESSION SANS ANTIOBIOTIQUES

(30) Priorität: 22.12.2003 DE 10360483
(43) Veröffentlichungstag der Anmeldung: 06.09.2006
(73) Patentinhaber: Scil proteins GmbH, 06120 Halle/Saale (DE)
(72) Erfinder: ANTON, Andreas, 06114 Halle (DE); FIEDLER, Markus, 06120 Halle (DE); FRINGS, Andreas, 06108 Halle (DE)
(74) Vertreter: Behnisch, Werner
(86) Internationale Anmeldenummer: PCT/EP2004/014635
(87) Internationale Veröffentlichungsnummer: WO 2005/061716

(56) Entgegenhaltungen:
- US-B1- 6 291 245
- GLENTING JACOB ET AL: "A plasmid selection system in Lactococcus lactis and its use for gene expression in L. lactis and human kidney fibroblasts" APPLIED AND ENVIRONMENTAL MICROBIOLOGY, Bd. 68, Nr. 10, Oktober 2002 (2002-10), Seiten 5051-5056, XP002331250 ISSN: 0099-2240
- FIEDLER M ET AL: "proBA complementation of an auxotrophic E. coli strain improves plasmid stability and expression yield during fermenter production of a recombinant antibody fragment" GENE: AN INTERNATIONAL JOURNAL ON GENES AND GENOMES, ELSEVIER SCIENCE PUBLISHERS, BARKING, GB, Bd. 274, Nr. 1-2, 22. August 2001 (2001-08-22), Seiten 111-118, XP004311057 ISSN: 0378-1119 in der Anmeldung erwähnt
- CHAO YUN-PENG ET AL: "Development of a fed-batch fermentation process to overproduce phosphoenolpyruvate carboxykinase using an expression vector with promoter and plasmid copy number controllable by heat." BIOTECHNOLOGY AND BIOENGINEERING, Bd. 84, Nr. 4, 20. November 2003 (2003-11-20), Seiten 459-466, XP002331251 ISSN: 0006-3592

### SEQUENCE LISTING

<110> Scil Proteins GmbH
<120> Expressionsvektor und dessen Verwendung
<130> P18474
<140> PCT/EP2004/014635
   <141> 2004-12-22
<150> DE10360483.9
   <151> 2003-12-22
<160> 14
<170> PatentIn version 3.3
<210> 1
   <211> 9
   <212> RNA
   <213> Escherichia coli
<400> 1
   uaaggaggu 9
<210> 2
   <211> 324
   <212> DNA
   <213> Artificial
<220>
   <223> synthetic MIA gene sequence
<400> 2
   atgggcccga tgccgaaact ggcggatcgt aaactgtgcg cggatcagga atgcagccat      60
   ccgattagca tggcggtggc gctgcaagat tacatggcgc cggattgccg ttttctgacc     120
   attcatcgtg gccaggtggt gtatgtgttt agcaaactga aaggccgtgg ccgtctgttt     180
   tggggcggca gcgtgcaggg cgattactat ggcgatctgg cggcacgtct gggctatttc     240
   ccgagcagca ttgtgcgtga agatcagacc ctgaaaccgg gcaaagtgga tgtgaaaacc     300
   gacaaatggg atttctattg ccag                                            324
<210> 3
   <211> 33
   <212> DNA
   <213> Artificial
<220>
   <223> Primer
<400> 3
   aaaaagcttg actcctgttg atagatccag taa 33
<210> 4
   <211> 31
   <212> DNA
   <213> Artificial
<220>
   <223> Primer
<400> 4
   aaaacatgta ttctcaccaa taaaaaacgc c 31
<210> 5
   <211> 25
   <212> DNA
   <213> Artificial
<220>
   <223> Primer
<400> 5
   aagggcccgc cacgttgtgt gtctc 25
<210> 6
   <211> 31
   <212> DNA
   <213> Artificial
<220>
   <223> Primer
<400> 6
   aaagctagcg atatcgccgt cccgtcaagt c 31
<210> 7
   <211> 28
   <212> DNA
   <213> Artificial
<220>
   <223> Primer
<400> 7
   aaagctagcg ggaataaggg cgacacgg 28
<210> 8
   <211> 30
   <212> DNA
   <213> Artificial
<220>
   <223> Primer
<400> 8
   aaagggccca cgtgagtttt cgttccactg 30
<210> 9
   <211> 29
   <212> DNA
   <213> Artificial
<220>
   <223> Primer
<400> 9
   aaagggcccg caaccgacga cagtcctgc 29
<210> 10
   <211> 29
   <212> DNA
   <213> Artificial
<220>
   <223> Primer
<400> 10
   aaagggcccc ggtggacaaa ggttaaaac 29
<210> 11
   <211> 27
   <212> DNA
   <213> Artificial
<220>
   <223> Primer
<400> 11
   aaagctagcg acaccatcga atggcgc 27
<210> 12
   <211> 25
   <212> DNA
   <213> Artificial
<220>
   <223> Primer
<400> 12
   aaagctagct cactgcccgc tttcc 25
<210> 13
   <211> 32
   <212> DNA
   <213> Artificial
<220>
   <223> Primer
<400> 13
   aaacaattgt gttgacaatt aatcatcggc tc 32
<210> 14
   <211> 35
   <212> DNA
   <213> Artificial
<220>
   <223> Primer
<400> 14
   aaagaattct ctccttgtga aattgttatc cgctc 35

## Beschreibung

Die vorliegende Erfindung betrifft einen Expressionsvektor zur Verwendung in einer auxotrophen, prokaryontischen Wirtszelle und betrifft ein Expressionssystem, das einen Expressionsvektor und eine auxotrophe, prokaryontische Wirtszelle enthält. Die Erfindung betrifft weiterhin ein antibiotikafreies Fermentationsmedium, das einen vorgenannten Expressionsvektor enthält sowie ein Verfahren zur antibiotikafreien Expression von Peptiden/Proteinen.

Die Verwendung von Expressionsvektoren, beispielsweise Plasmiden zur Expression beispielsweise von therapeutischen Peptiden/Proteinen ist seit langem bekannt. So hat es die Produktion rekombinanter Proteine in großem Maßstab beispielsweise in *E. coli* möglich gemacht, ein breites Spektrum von Proteinen für die biochemische Forschung, die Biotechnologie und sogar für die medizinische Therapie verfügbar zu machen. Ein Beispiel für die erfolgreiche Anwendung dieser Methodologie ist die bakterielle Produktion eines Antigen-bindenden Immunglobulin F_{ab}-Fragmentes bei mittleren und hohen Zelldichten (Carter *et al.,* 1992; Schiweck und Skerra, 1995). Im allgemeinen hat die heterologe Biosynthese, die auf die gentechnische Herstellung von therapeutischen Proteinen gerichtet ist, während des letzten Jahrzehntes zunehmend an Interesse gewonnen.

Ein Problem, das oftmals bei der Produktion im industriellen Maßstab zu beobachten ist, ist die Abnahme der Ausbeute an rekombinantem Protein pro Zelle. Ein Grund für dieses Phänomen ist der Verlust von funktionellem Plasmid in Kulturen mit hoher Zelldichte, der auf eine aufspaltende oder strukturelle Instabilität genetisch sicherer Expressionsvektoren zurückzuführen ist (Corchero und Villaverde, 1998). Im Gegensatz zu natürlichen Vektoren, die ColEI tragen, fehlen solchen Plasmiden Mobilitäts- und Verteilungs-Funktionen, die normalerweise die Vererbung und die Nachkommenschaft sicherstellen. Im Labor wird die reduzierte genetische Stabilität durch eine Antibiotika-Selektion der Bakterien unter Verwendung eines Resistenzmarkers kompensiert. Es ist jedoch schwierig, diesen Selektionsdruck unter Fermenter-Bedingungen aufrecht zu erhalten und ein Verlust an funktionellem Plasmid kann somit stattfinden (Zabriskie und Arcuri, 1986; Broesamle *et al.,* 2000)*.*

Ein weiterer bedeutender Parameter für die erfolgreiche Produktion eines rekombinanten Proteins in hoher Ausbeute ist die Auswahl eines adäquaten bakteriellen Wirtsstammes, der die Konzentration des synthetisierten Genproduktes wesentlich beeinflussen kann (Sambrook *et al.,* 1989). Einer von diesen ist beispielsweise der *E. coli* K12 Stamm JM83, der für mehrere Jahre im Labormaßstab für die Expression von Antikörperfragmenten über die periplasmatische Sekretion erfolgreich verwendet wurde (Skerra *et al.,* 1991; Fiedler und Skerra, 1999). Bei dem *E. coli* K12 Stamm JM83 handelt es sich um ein Beispiel für einen auxotrophen Stamm, d.h., dem Stamm fehlt die Fähigkeit, eine essentielle Aminosäure aus eigenem Vermögen zu erzeugen. Im Falle von *E. coli* K12 Stamm JM83 ist dies die Aminosäure Prolin. Dieser Wirtsstamm zeigt eine überlegenere funktionelle Expression im Vergleich mit vielen anderen *E. coli* Stämmen, wenn das Protein in das bakterielle Periplasma sezerniert wird (Skerra 1994 a, b). Jedoch war dieser Prolinauxotrophe Stamm für Fermentationsexperimente wegen seiner Unfähigkeit, in einem minimalen Medium zu wachsen, nicht geeignet. Die Verwendung synthetischer Medien zur Fermentation wird im industriellen Produktionsmaßstab im allgemeinen bevorzugt, weil die Wachstumseigenschaften in einfacher Weise unter Verwendung einer definierten Kohlenstoffquelle, in den meisten Fällen in Glucose oder Glycerol, kontrolliert werden können (Yee und Blanch, 1992). Jedoch erwies sich im Falle von JM83 eine Ergänzung eines Glucose/Ammoniak/Mineralsalzmediums mit Prolin als nicht durchführbar, weil diese Aminosäure vorzugsweise sowohl als Kohlenstoff- als auch als Stickstoff-Quelle metabolisiert wird (Neidhard *et al.,* 1990) und daher die Wachstumsgeschwindigkeit bestimmt.

Der *pro*BA-Lokus (Mahan und Csonka, 1983; Deucht *et al.,* 1984) kodiert γ-Glutamylkinase (ProB) und Glutamat-5-semialdehyddehydrogenase (ProA), die beide im Prolin-Biosyntheseweg eine Schlüsselrolle spielen.

M. Fiedler und A. Skerra offenbaren in Gene 274 (2001) 111-118 einen Expressionsvektor, genauer ein Plasmid, das folgende Elemente in funktioneller Verbindung umfasst: einen Repressor zur Kontrolle der Expression (TetR), einen Promotor für die Expression (*tet*)*,* ein Antibiotikaresistenzgen für Chloramphenicol sowie *proBA.* Die vorgenannte Veröffentlichung beschreibt jedoch nicht, dass das Plasmid unter Verwendung der Aminosäure als einzigem Selektionsmittel und einzigem Marker einsetzbar ist. Weiterhin offenbaren Fiedler *et al.* nicht den Einsatz des vorgenannten Plasmids zur Produktion rekombinanter Proteine in einem antibiotikafreien Fermentationsmedium. Insbesondere wird des Einsatz des tac-Promotors nicht offenbart.

Der Einsatz von Antibiotika zur Selektion von Plasmiden ist jedoch ein entscheidender Nachteil bei der Herstellung insbesondere therapeutischer Proteine. Ein antibiotikafreies Verfahren würde eine größere Akzeptanz seitens der Regulierungsbehörden (z.B. FDA, EMEA) mit sich bringen, da das Produkt sicherer für den Patienten sein wird und man auf Grund der geringen Endproduktanalytik (Abreicherung des Antibiotikums im Produkt) einen deutlich preiswerteren Prozess konstruieren kann.

Der vorliegenden Erfindung liegt somit die Aufgabe zugrunde, ein Verfahren zur antibiotikafreien Expression von Peptiden/Proteinen bereitzustellen. Der Erfindung liegt die weitere Aufgabe zugrunde, ein zur Verwendung in diesem Verfahren geeignetes Fermentationsmedium und Expressionssystem bereitzustellen.

Diese Aufgaben werden durch den Gegenstand der unabhängigen Ansprüche gelöst. Bevorzugte Ausführungsformen sind in den Unteransprüchen angegeben.

Kernpunkt der vorliegenden Erfindung ist die Generierung eines Expressionsystems, das eine antibiotikafreie Fermentation, d.h. Protein/Peptid-Expression ermöglicht.

Die vorliegende Erfindung ist insbesondere auf Folgendes gerichtet:

Gemäß einem ersten Aspekt betrifft die Erfindung einen Expressionsvektor zur Verwendung in einer auxotrophen, prokaryontischen Wirtszelle, der die folgenden Bestandteile in funktioneller Verbindung umfasst:
a) eine regulatorische Sequenz,
b) eine für ein Protein/Peptid kodierende Sequenz,
c) ein erstes selektierbares Markergen, und
d) ein zweites selektierbares Markergen, wobei das Markergen ein vom auxotrophen Wirt nicht exprimiertes Protein kodiert, welches für die Biosynthese einer Aminosäure, in der die Wirtszelle auxotroph ist, notwendig ist, und wobei das zweite selektierbare Markergen proBA ist, und
es sich bei der regulatorischen Sequenz um einen *tac*-Promotor mit einer Ribosomenbindestelle handelt.

Gemäß einer Ausführungsform weist der erfindungsgemäße Expressionsvektor einen Terminator zur Termination der Transkription auf, wobei besonders bevorzugt der Terminator *tₒ* aus dem Bakteriophagen Lambda eingesetzt wird. Letztendlich kann anstelle dessen in dieser Position jeder funktionelle Terminator eingesetzt werden. Hierfür existieren zahlreiche Beispiele da die meisten Operons oder Gene mit einer Temninatorstruktur flankiert werden, um ein Durchlesen der Polymerase zu verhindern.

Der erfindungsgemäße Expressionsvektor weist weiterhin ein Repressorgen auf, bei dem es sich besonders bevorzugt um ein *lacI*-Gen handelt. Ein Repressorgen, wie hierin verwendet, umfasst jedes Gen, das ein Protein kodiert, das bei Bindung im Promotorbereich die Transkription von Genen verhindert. Als Beispiel hierfür dient erfindungsgemäß das oben genannte *lac*-Repressorgen (insbesondere das *lac*-Repressorgen aus *E*. *coli* K12; siehe Beispiele).

Ein weiterer bekannter Repressor ist der CI-Repressor aus dem Phagen Lambda, der jedoch nicht an die *lac*-Hybrid-Promotoren sondern nur an die Promotoren P_{L} und P_{R} bindet. Auch der AraC-Repressor kann verwendet werden, da er die Transkription vom *pBAD* (*ara*) Promotor unterbindet. Dem Fachmann sind jedoch zahlreiche weitere Beispiele für entsprechend einsetzbare Repressoren bekannt.

Sequenzinformationen hierzu sind beispielsweise in folgenden Literaturstellen zu finden
- für CI:
   Sauer RT, DNA sequence of the bacteriophage gama cI gene. Nature. 1978 Nov 16;276(5685):301-2.
   Humayun,Z. DNA sequence at the end of the CI gene in bacteriophage lambda, Nucleic Acids Res. 4 (7), 2137-2143 (1977)
   Sequenzinformation und zusätzliche Literatur des Repressors zu finden unter:
   http://www.ncbi.nlm.nih.gov/entrez/viewer.fcgi?val=9626243&itemID=48&view= gbwithparts
- für P_{L} und P_{R}:
   Horn, G.T. and Wells,R.D. The leftward promoter of bacteriophage lambda. Isolation on a small restriction fragment and deletion of adjacent regions, J. Biol. Chem. 256 (4), 1998-2002 (1981)
   Remaut,E., Stanssens,P. and Fiers,W. Plasmid vectors for high-efficiency expression controlled by the PL promoter of coliphage lambda, Gene 15 (1), 81-93 (1981)
   Petrov,N.A., Karginov,V.A., Mikriukov,N.N., Serpinski,O.I. and Kravchenko, V.V. Complete nucleotide sequence of the bacteriophage lambda DNA region containing gene Q and promoter pR', FEBS Lett. 133 (2), 316-320 (1981)
   Walz,A. and Pirrotta, V. Sequence of the PR promoter of phage lambda; Nature 254 (5496), 118-121 (1975)
   Sequenzinformationen der Promotoren sind zu finden unter:
      http://www.ncbi.nlm.nih.gov/entrez/query.fcgi?cmd=Retrieve&db=nucleotide&list uids=14819
      http://www.ncbi.nlm.nih.gov/entrez/query.fcgi?cmd=Retrieve&db=nucleotide&list uids=341294
      http://www.ncbi.nlm.nih.gov/entrez/query.fcgi?cmd=Retrieve&db=nucleotide&list uids=215104
- für AraC:
   Miyada,C.G., Horwitz,A.H., Cass,L.G., Timko,J. and Wilcox,G. DNA sequence of the araC regulatory gene from Escherichia coli B/r, Nucleic Acids Res. 8 (22), 5267-5274 (1980)
   Wallace,R.G., Lee,N. and Fowler,A.V. The araC gene of Escherichia coli: transcriptional and translational start-points and complete nucleotide sequence, Gene 12 (3-4), 179-190 (1980)
   Sequenzinformationen des AraC sind zu finden unter:
      http://www.ncbi.nlm.nih.gov/entrez/query fcgi?cmd=Retrieve&db=nucleotide&list uids=40935
   für *pBAD*-Promotor:
   Miyada,C.G., Sheppard,D.E. and Wilcox,G. Five mutations in the promoter region of the araBAD operon of Escherichia coli B/r, J. Bacteriol. 156 (2), 765-772 (1983)
   Sequenzinformationen des araBAD-Promotors sind zu finden unter:
      http://www.ncbi.nlm.nih.gov/entrez/query.fcgi?cmd=Retrieve&db=nucleotide&list uids=145308

Gemäß einer bevorzugten Ausführungsform enthält der erfindungsgemäße Expressionsvektor eine Ribosomenbindestelle mit der Sequenz AGGAGA.

Bei einer ribosomalen Bindungsstelle bzw. Ribosomenbindungsstelle handelt es sich um eine Bindestelle für die kleine Untereinheit des Ribosoms auf der *m*RNA stromaufwärts des Startkodons. Bei Prokaryonten ist dies allgemein die Shine-Dalgamo-(SD-) Sequenz. Sie liegt häufig drei bis elf Nukleotide flussaufwärts des Startkodons und zeigt Komplementarität zu einem Bereich am 3' Ende der 16sRNA. Die *Escherichia coli* SD-Konsensus Sequenz ist UAAGGAGGU.

Gemäß einer weiteren Ausführungsform des Expressionsvektors der Erfindung ist das erste selektierbare Markergen ein Antibiotikaresistenzgen, vorzugsweise ein Kanamycinresistenzgen. Im vorliegenden Kontext einsetzbare Antibiotikaresistenzgene sind z.B.: Ampicillin-Resistenz (amp); Tetrazyklinresistenz (tet); Chloramphenicolresistenz (cat); Neomycinresistenz (gleiches Resistenzgen wie Kanamycin; z.B. das Km-Resistenzgen aus dem Vektor pACYC177; siehe Beispiele).

Informationen zu pACYC177 sind unter: Rose, R.E. The nucleotide sequence of pACYC177, Nucleic Acids Res. 16 (1), 356 (1988), sowie unter dem Link http://www.fermentas.com/techinfo/nucleicacids/sequences/pacyc177.txt zu finden.

Der erfindungsgemäße Expressionsvektor enthält, wie oben beschrieben, ein zweites selektierbares Markergen, wobei das Markergen eine vom auxotrophen Wirt nicht exprimierte Aminosäure kodiert. Dieses zweite, selektierbare Markergen wird hierin auch als Auxotrophiegen bezeichnet. Das zweite selektierbare Marker ist *proBA*.

Folgende Literaturdaten zu *proBA* beschreiben die Gene und deren Aktivität: Baich, A., (1969) Proline synthesis in Escherichia coli. A proline inhibitable-glutamic acid kinase. Biochim Biophys Acta 192, 462-467. Baich, A., (1971) The biosynthesis of proline in Escherichia coli, phosphate-dependent glutamate γ-semialdehyde dehydrogenase (NADP), the second enzyme in the pathway. Biochim Biophys Acta 244, 129-134.

Durch den erfindungsgemäßen Expressionsvektor können zahlreiche kodierende Sequenzen exprimiert werden, deren Auswahl an sich keinen Beschränkungen unterliegt. Insbesondere hat sich der erfindungsgemäße Expressionsvektor zur Expression für G-CSF, MIA und/oder BMP kodierenden Sequenzen als vorteilhaft herausgestellt. Weiterhin können alle therapeutisch relevanten Proteine (z.B. tPA, TNF, HGF, NGF, Proteasen wie Trypsin, Thrombin, Enterokinase, β-TGF, Interferone, Erythropoietin, Insulin, Faktor VII, Faktor VIII, Single Chain Antikörper, Affilin^{™} sowie Fusionen dieser Proteine, G-Protein-gekoppelte Rezeptoren sowie deren Domänen, sowie die Proformen dieser Proteine) die als *Inclusion Bodies* oder als lösliche Varianten gebildet werden sollen, exprimiert werden. Zuletzt ist auch die Expression von GM-CSF, M-CSF, Interleukinen, Interferonen, Calcitonin, Caspasen, VEGF, Factor III, Factor X, Factor Xa, Factor XII, Factor XIIa, GDF, IGF, Metalloproteasen, Antikörpern, Antikörperfragmenten oder Immunotoxinen denkbar.

Gemäß einer weiteren Ausführungsform handelt es sich bei dem erfindungsgemäßen Vektor um ein *high copy* Plasmid. Bei einem *high-copy*-plasmid bzw. *high-copy-number*plasmid (auch multi-copy-plasmid genannt) handelt es sich um kleine Plasmide (in der Regel < 15kb) mit hoher Kopienzahl (> 20 Plasmide/ Chromosom). Derartige Plasmide, wie beispielsweise von pBR322 abgeleitete pUC Plasmide werden häufig als Klonier- und Expressionsvektoren eingesetzt.

In einer speziellen Ausführungsform enthält der Expressionsvektor der Erfindung die folgenden Bestandteile in funktioneller Verbindung:
a) einen *tac*-Promotor mit einer Ribosomenbindestelle,
b) eine kodierende Sequenz für z.B. MIA,
c) ein Kanamycinresistenzgen,
d) eine *proBA*-Sequenz,
e) wahlweise das *lacI*-Repressorgen, und
f) wahlweise den *tₒ*.Terminator aus Lambda zur Beendigung der Transkription eines Gens.

Weiterhin betrifft die Erfindung den Expressionsvektor pSCIL008, der folgende Bestandteile in funktioneller Verbindung enthält:
a) einen *tac*-Promotor mit einer Ribosomenbindestelle,
b) eine kodierende Sequenz für z.B. MIA, G-CSF, ProBMP, BMP, tPA, TNF, HGF, NGF, Proteasen wie Trypsin, Thrombin, Enterokinase, β-TGF, Interferone, Erythropoietin, Insulin, Faktor VII, Faktor VIII, Single Chain Antikörper, Affilin^{™} sowie Fusionen dieser Proteine, G-Protein-gekoppelte Rezeptoren sowie deren Domänen, sowie die Proformen dieser Proteine, GM-CSF, M-CSF, Interleukine, Interferone, Calcitonin, Caspase, VEGF, Factor III, Factor X, Factor Xa, Factor XII, Factor XIIa, GDF, IGF, Metalloproteasen, Antikörper, Antikörperfragmente, Immunotoxine,
c) ein Antibiotikaresistenzgen, vorzugsweise ein Kanamycinresistenzgen,
d) eine *proBA*-Sequenz,
e) wahlweise ein an den Operator des Promotors bindender Repressor, vorzugsweise das *lacI*-Repressorgen, und
f) wahlweise den *tₒ*.Terminator aus Lambda zur Beendigung der Transkription eines Gens.

Gemäß einem zweiten Aspekt betrifft die vorliegende Erfindung ein Expressionssystem, das die folgenden Bestandteile umfasst:
a) einen Expressionsvektor wie hierin definiert, und
b) eine auxotrophe prokaryontische Wirtszelle.

Erfindungsgemäß handelt es sich bei der Wirtszelle vorzugsweise um eine auxotrophe *E. coli*-Zelle, die in der Aminosäure Prolin auxotroph ist.

Die *E*. *coli*-Zelle ist vorzugsweise aus den Stämmen JM106, JM108, JM109, JM83 und TB1 oder deren Derivaten ausgewählt. Derivate bedeuten Stämme, die genetisch manipuliert wurden, und die für die Expression relevante Auxotrophien behalten und somit auch mit den erfindungsgemäßen Vektoren, die Aminosäureauxotrophien komplementieren, transformiert werden können. Von der Erfindung mit umfasst sind auch solche Stämme, die mit Verfahren nach dem Stand der Technik soweit manipuliert wurden, dass sie Auxotrophien erhalten, die man als Selektionsmarker benutzen kann.

Die oben genannten *E. coli* Stämme sind unter folgenden DSMZ-Nummer hinterlegt worden:
JM83 DSMZ-Nr. 3947
JM108 DSMZ-Nr. 5585
JM109 DSMZ-Nr. 3423
JM106 wird ausführlich in Yanisch-Perron *et al.,* 1985 beschrieben. Informationen zu TB-1 sind in Biochemistry 23:3663-3667 1984 zu finden.

Gemäß einem dritten Aspekt betrifft die Erfindung ein antibiotikafreies Fermentationsmedium, das ein Expressionssystem wie oben definiert umfasst.

Das Fermentationsmedium umfasst bei Vorliegen eines Repressorgens wahlweise einen Induktor.

Gemäß einem vierten Aspekt betrifft die vorliegende Erfindung ein Verfahren zur antibiotikafreien Expression von Peptiden/Proteinen, das die folgenden Schritte umfasst:
a) Transformieren von auxotrophen Wirtszellen mit einem Expressionsvektor wie hierin definiert,
b) Selektion auf transformierte Wirtszellen, wobei die Selektion aufgrund der durch das zweite selektierbare Markergen exprimierten Aminosäure erfolgt;
c) Einbringen der transformierten Wirtszellen in ein Fermentationsmedium wie oben erläutert unter solchen Bedingungen, dass eine Fermentation stattfindet und das Protein/Peptid exprimiert wird; und
d) Isolieren und Aufreinigen des exprimierten Proteins/Peptids.

Mit anderen Worten erfolgt bei der vorliegenden Erfindung insbesondere der Fermentationsprozess, d.h. der eigentliche Verfahrensabschnitt zur Expression der Peptide/Proteine, in einer vollkommen antibiotikafreien Umgebung. Dadurch können die eingangs genannten Probleme, die mit dem Einsatz von Antibiotika zur Selektion von Plasmiden verbunden sind, beispielsweise Vorbehalte der Regulierungsbehörden, Produktsicherheit, Endproduktanalytik (Abreicherung des Antibiotikums im Produkt) und die damit verbundenen Risiken und Kosten, umgangen werden. Durch den erfindungsgemäßen Ansatz bleibt der Selektionsdruck im Fermentationsverfahren also aufrecht erhalten und dies ohne den Einsatz von Antibiotika im Fermentationsmedium.

Die vorliegende Erfindung wird nunmehr anhand von Zeichnungen und der beigefügten Ausführungsbeispiele näher erläutert. Es sollte klar sein, dass der Umfang der Erfindung nicht auf diese Ausführungsformen beschränkt ist und diese lediglich zu Veranschaulichungszwecken dienen sollen.

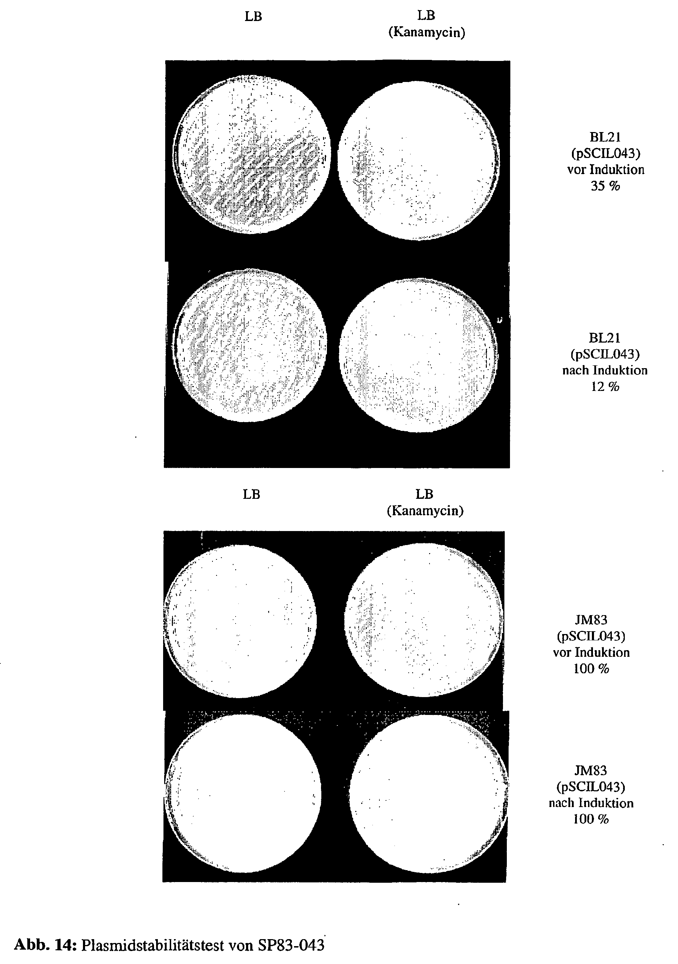

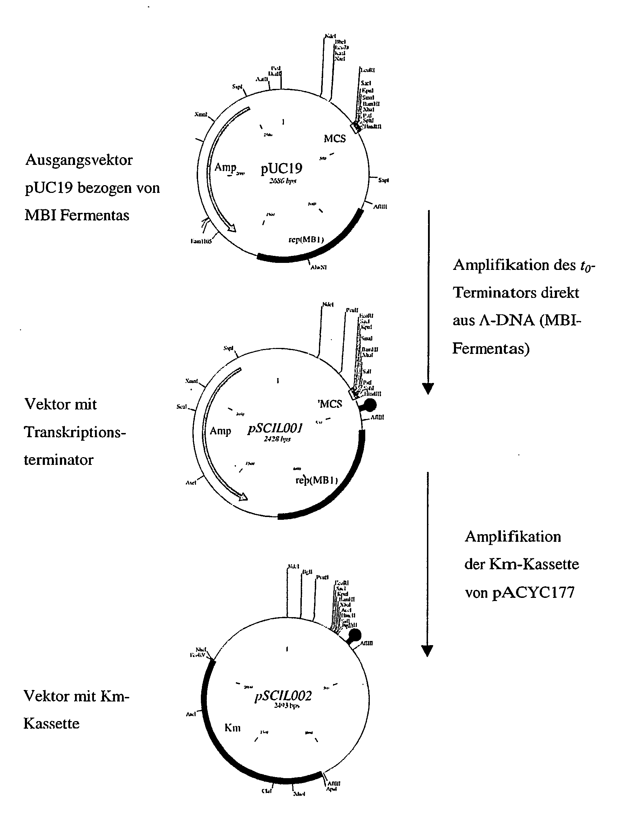

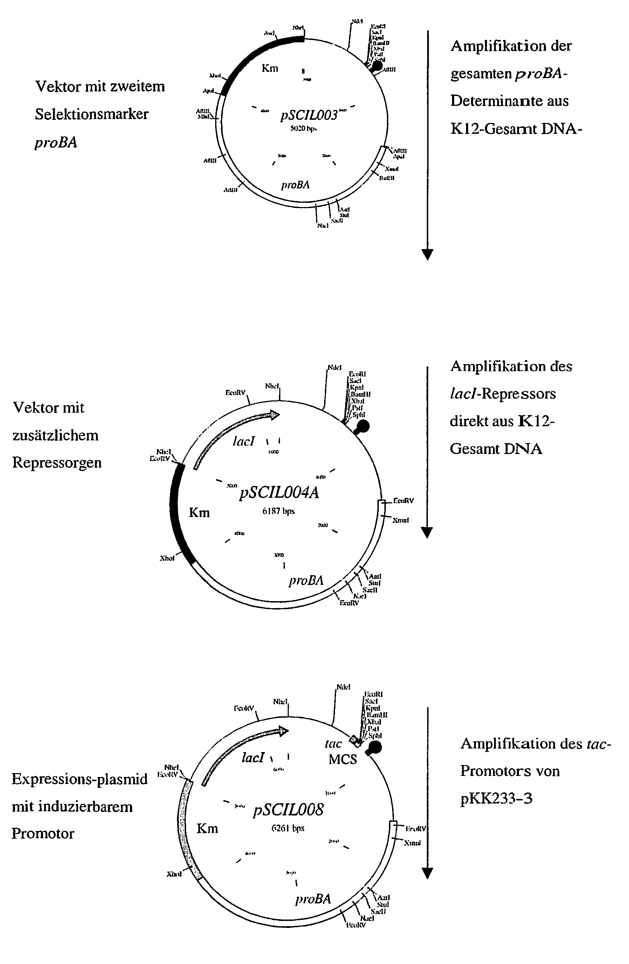

In den Abbildungen werden die Schritte zur Konstruktion von pSCIL043 dargestellt:
**Abb.1:** Eine Plasmidkarte des pSCIL043-Expressionsvektors.
   Alle relevanten Genbereiche wurden farbig hervorgehoben. Folgende Bereiche sind für die Funktion des Vektors wichtig: *lacI* (Repressor, rot); *Km* (Antibiotikaresistenz, grün); *proBA* (Selektionsmarker, gelb); *t*₀ (Terminator, dunkelgrün); *tac* (Promotor, blau).
**Abb.2:** Restriktion von pUC 19 mit *Afl*III und *Hin*dIII
   Durch diese Restriktion werden nicht kodierende Plasmidbereiche aus pUC19 deletiert und es entstehen zwei Fragmente: ein für die Funktion nicht relevantes Fragment von 359 bp Größe und ein 2327 bp großes Fragment, das den restlichen Vektor repräsentiert. Das 2327 bp große Fragment wurde gereinigt und weiterveiwendet. **1**: 100 bp Marker Invitrogen; **2**: pUC19 *Afl*III/*Hin*dIII-Restriktion; **3**: pUC19 ungeschnitten; **4**: 1 Kb Marker MBI.

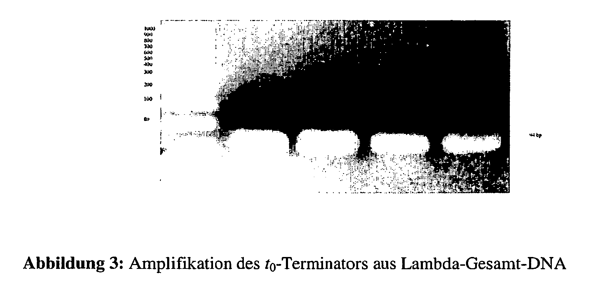

**Abb.3**: Amplifikation des *t₀*-Terminators aus Lambda-Gesamt-DNA
   Direkt aus der chromosomalen Lambda-DNA, bezogen von MBI-Fermentas, wurde *der t₀* Terminator (94 bp) mittels PCR amplifiziert.

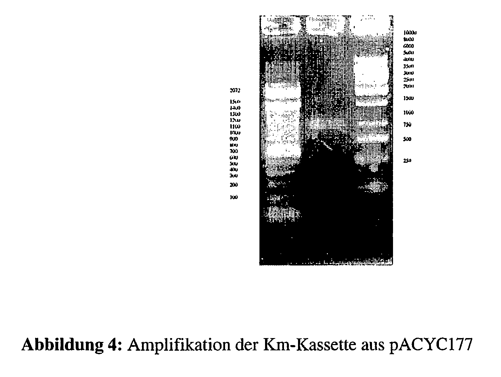

**Abb.4**: Amplifikation der Km-Kassette aus pACYC177
   Als Template für die Km-Kassette wurde pACYC177 verwendet. Das PCR-Produkt wurde nach der Reinigung durch das Gel Extraktion Kit (Qiagen) in pGEM-Teasy (Promega) zwischenkloniert.

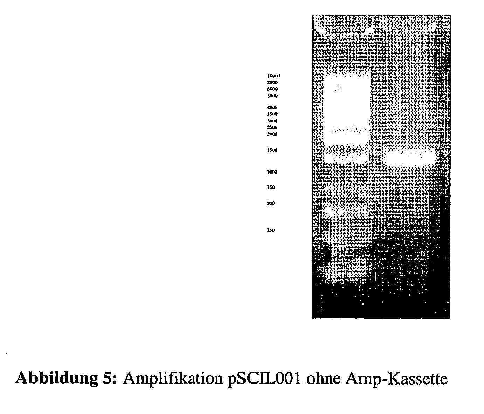

**Abb.5**: Ampliflkation pSCIL001 ohne Amp-Kassette
   Um die Antibiotika-Resistenzen auszutauschen sowie um zwei neue Restriktionsschnittstellen (*Nhe*I und *Apa*I) einzuführen, wurde pSCIL001 mittels die Amp-Kassette flankierenden Primern amplifiziert.

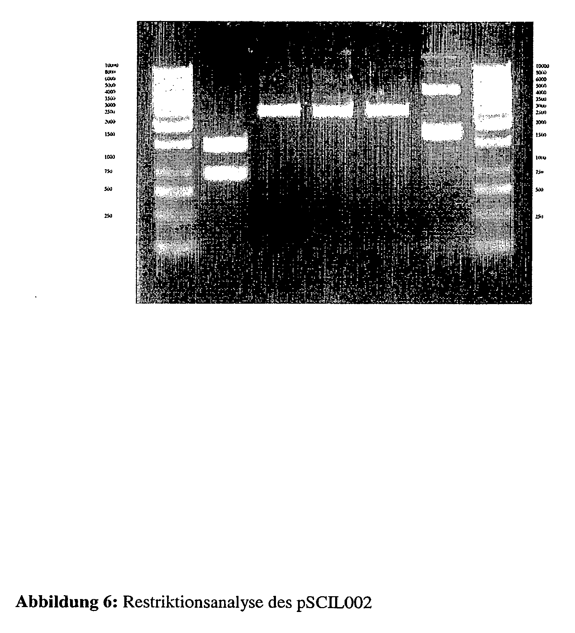

**Abb.6**: Restriktionsanalyse des pSCIL002
   Durch die Restriktion mit *Apa*I/*Nhe*I wird die Km-Kassette wieder aus dem Vektor geschnitten. Bei den Restriktionen mit *Eco*RI*, Eco*RV und *Afl*III wurde der Vektor nur linearisiert.**1**: 1 kb Marker MBI; **2:** pSCIL002 *Apa*I/*Nhe*I*;* **3:** pSCIL002 *Eco*RI; **4:** pSCIL002 *Eco*RV; **5:** pSCIL002 *Afl*III.

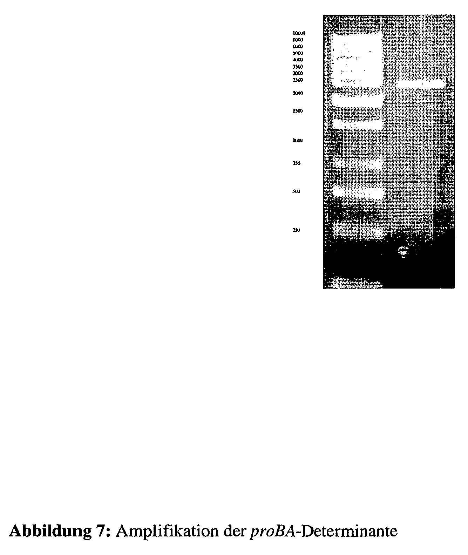

**Abb.7**: Amplifikation der *proBA*-Determinante
   Mittels der oben genannten Primer konnte die *proBA*-Determinante amplifiziert werden. Das Fragment enthält den nativen Terminator der *proBA* Gene.

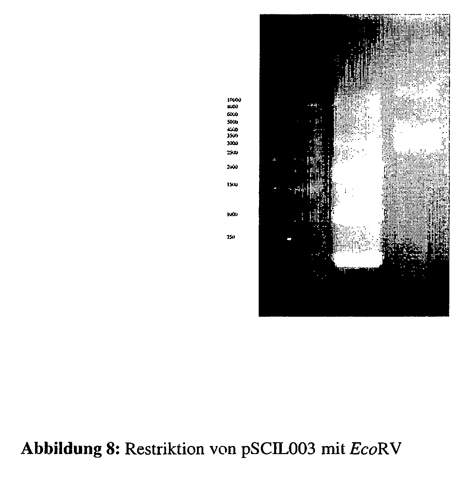

**Abb.8:** Restriktion von pSCIL003 mit *Eco*RV
   Zur Überprüfung des richtigen Einbaus der *proBA*-Determinante in pSCIL002 wurde das entstandene Plasmid mit *Eco*RV geschnitten, dabei sollten folgende Fragmente entstehen: 2479, 1542 und 999 bp. Bei einem negativen Ergebnis mit pSCIL002 sollte der Vektor nur linearisiert werden. 1: 1 kb-Marker MBI; 2: pSCIL003 *Eco*RV*;* 3: pSCIL003 ungeschnitten.

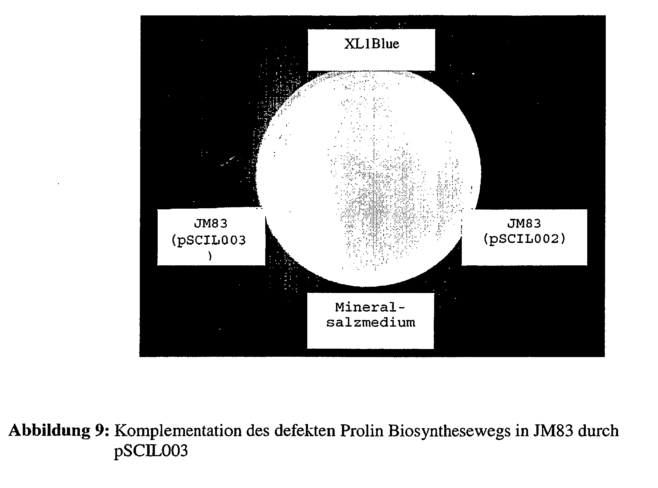

**Abb. 9**: Komplementation des defekten Prolin Biosynthesewegs in JM83 durch pSCIL003
   Auf Vollmedium wachsen alle Sämme (Wildtyp = XLlBlue) genauso wie der komplementierte Stamm (nicht gezeigt). Auf Mineralsalzmedium ist es aber nur dem Stamm mit pSCIL003, nicht aber mit pSCIL002 möglich zu wachsen.

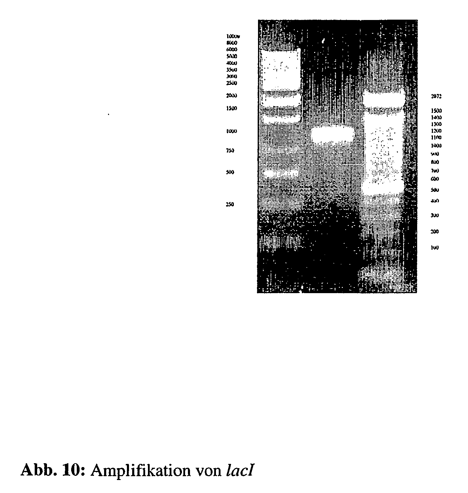

**Abb. 10:** Amplifikation von *lacI*
   Mittels der oben genannten Primer konnte der Repressor *lacI* direkt aus der chromosomalen K12 DNA amplifiziert werden. Das Gen wurde in den Zwichenklonierungsvektor pGEM Teasy (Promega) ligiert und die Integrität des PCR-Produktes durch Restriktionsanalyse und Sequenzierung überprüft.

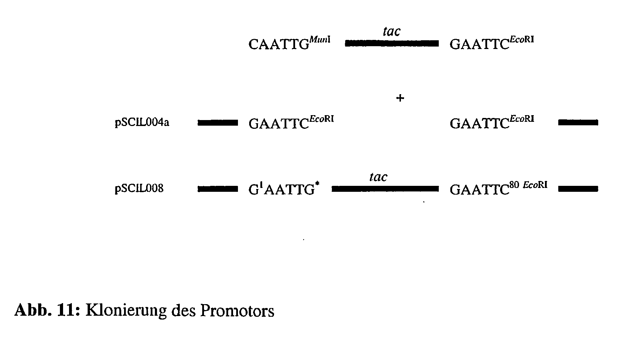

**Abb. 11**: Klonierung des Promotors
   Durch das PCR-Produkt wurde eine der beiden *Eco*RI-Schnittstellen bei der Ligation zerstört.

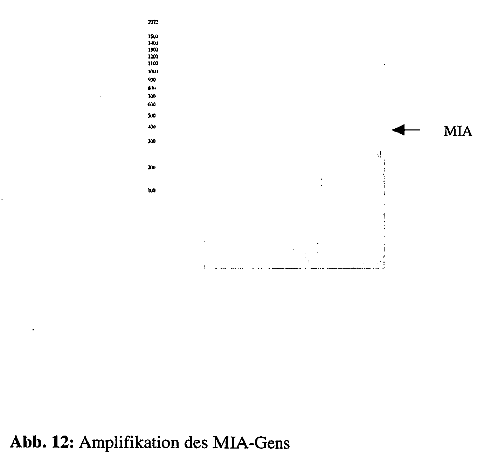

**Abb. 12**: Klonierung des MIA-Gens
   Aus dem Syntheseprodukt des MIA-Gens wurde mittels PCR amplifiziert. Nach Zwischenklonierung in pGEM-Teasy wurde das Fragment nach einer Restriktion über eine Agarosegelelektrophorese aufgetrennt und gereinigt. Anschließend wurde das DNA-Fragment in pSCIL008^{*Eco*RI/*Pst*I} ligiert.

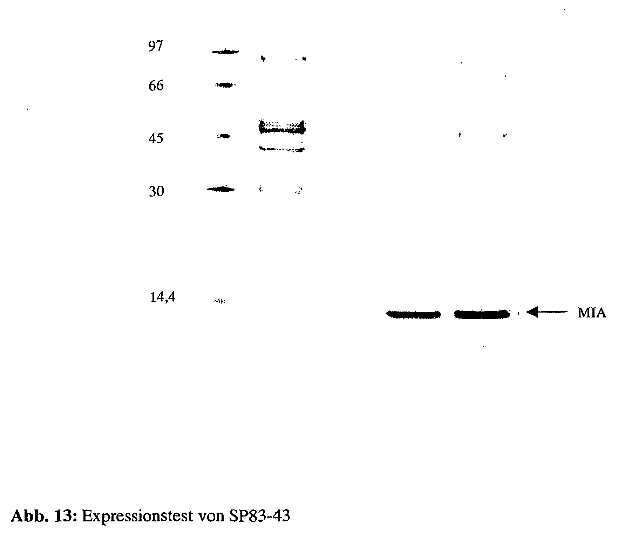

**Abb. 13**: Expressionstest von SP83-043 (JM83 [pSCIL043])
   Der Expressionstest mit pSCIL043 wurde mit JM83 in Mineralsalzmedium durchgeführt. Bei einer OD von 0.8 wurde mit 1 mM IPTG induziert. Dargestellt ist die Expression vor Induktion sowie in den Stunden nach Induktion **1**: Marker **2**: SP83-043 (MSM) vor Induktion; **3**: SP83-043 (MSM) 1 h nach Induktion; **4**: SP83-043 (MSM) 2 h nach Induktion; **5**. SP83-043 (MSM) 3 h nach Induktion
**Abb. 14**: Plasmidstabilitätstest von SP83-043
   Nach dem Ende der Expression wurden die Zellen verdünnt auf festem LB-Medium ausplattiert. Die gewachsenen Kolonien wurden auf LB-Agar ohne Antibiotika und mit Antibiotika gepickt. Alle Kolonien wuchsen auf beiden Medien. Daraus ergibt sich eine Plasmidstabilität von 100 %. Im Vergleich dazu wurde bei einem parallel durchgeführten Expressionstest in *E*. *coli* BL21 eine Plasmidstabilität von 12-35 % ermittelt.

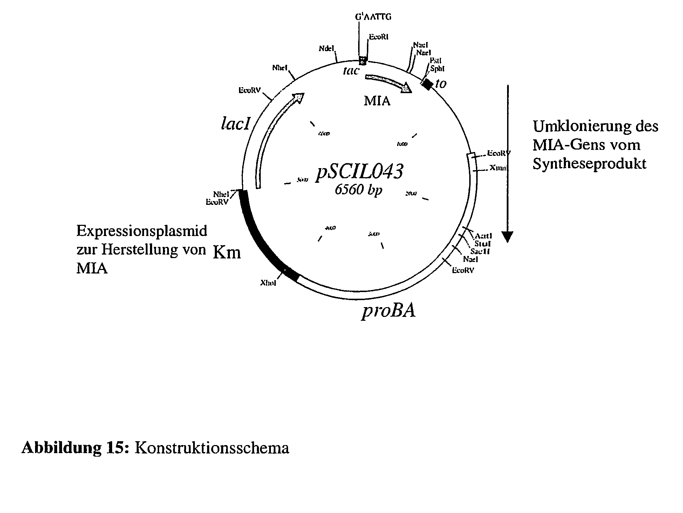

**Abb. 15:** Konstruktionsschema von pSCIL043

### Beispiele:

### Beispiel 1: Beschreibung der Zelllinie SP83-043 [JM83(pSCIL043]

### 1) Herkunft, Phänotyp und Genotyp des Expressionsstammes

Der bakterielle Wirt *Escherichia coli* JM83, der zur Expression von MIA verwendet wird, wurde von der Deutschen Sammlung für Mikroorganismen und Zellkulturen GmbH, Braunschweig (DSMZ) bezogen und ist durch ein Zertifikat identifiziert.

Der verwendete *E. coli* Stamm JM83 (DSMZ 3947) [F⁻, *ara,* Δ(*lac-proAB*), *rpsL* (Str^{r}), φ*80lacZ*Δ*M15, thi*] ist aufgrund einer Mutation im *rpsL-*Gen (12 S Protein der 30 S Untereinheit des bakteriellen Ribosoms) gegenüber Streptomycin resistent. Der Stamm ist nicht in der Lage, aufgrund einer Mutation im *proBA-*Operon Prolin zu synthetisieren. Dieser Effekt wird durch den Einsatz von pSCIL043 allerdings aufgehoben und diese Auxotrophie wird als Selektionsmarker genutzt. Weiterhin kann der Stamm keine Arabinose metabolisieren und ist wie viele andere K12-Derivate (z.B. C600; DH5α, JM107) nicht in der Lage Thiamin zu synthetisieren (Vieira & Messing, 1982).

### 2) Expression von MIA

Das Protein MIA wird im *Escherichia coli* Stamm JM83 unter Kontrolle des *tac*-Promotors, der auf pSCIL043 lokalisiert ist, exprimiert. Der Vektor pSCIL043 ist ein *high copy* Plasmid mit einer Kanamycin Resistenz. Die Expression erfolgt in definiertem Mineralsalzmedium und wird durch die Zugabe von IPTG induziert. Das MIA wird in Form von Einschlußkörpern, sogenannten *Inclusion bodies* synthetisiert.

### 3) Referenzen

Vieira, J. und Messing, J. (1982); Gene 19, S. 259
JM83 (DSMZ-Nr. 3947)
MIA-Gensequenz (synthetisches Gen):

### Detaillierte Beschreibung des Expressionsvektors pSCIL043

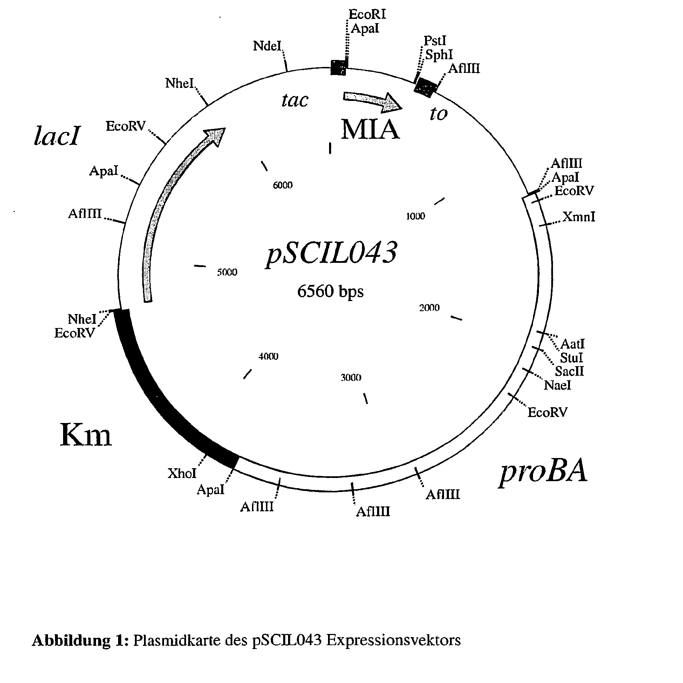

Das Plasmid pSCIL043 wird im folgenden detailliert beschrieben. Als erste Base wird die durch die Klonienung nicht mehr intakte *Mun*I/*Eco*RI*-*Site (G¹AATTG) angesetzt. Eine Plasmidkarte ist in Abbildung 1 abgebildet:

| Position (bp) | Funktion/Beschreibung |
|---|---|
| 1-730 | für Expression relevanter Bereich |
| 7-74 | • *tac*-Promotor inklusive RBS (AGGAGA) |
| 75-80 | • *Eco*RI-Schnittstelle (verwendet für Klonierung) |
| 83-406 | • hMIA-Gen |
| 407-412 | • TAATGA Stop Codons |
| 413-418 | • *Pst*I-Schnittstelle (verwendet für Klonierung) |
| 425-430 | • *Hin*dIII-Schnittstelle (verwendet für Klonierung) |
| 431-525 | • *t₀*-Terminator des Bakteriophagen Lambda |
| 526-531 | • *Afl*III-Schnittstelle (verwendet für Klonierung) |
| 532-1232 | Replikationsorigin pUC19 |
| 1233-5929 | Selektionsmarker |
| 1233-1238 | • *Apa*I-Schnittstelle (verwendet für Klonierung) |
| 1239-3759 | • *proBA*-Determinante als *Apa*I/*Apa*I-Fragment |
| | (Selektionsmarker) aus *E coli* K12 |
| 3760-3765 | *• Apa*I*-* Schnittstelle (verwendet für Klonierung) |
| 3766-4756 | • Km-Resistenzgen aus dem Vektor pACYC177 |
| 4757-4762 | • *Nhe*I-Schnittstelle (verwendet für Klonierung) |
| 4763-5923 | *lac*I-Repressorgen als *Nhe*I-Fragment aus *E coli* K12 |
| 5924-5929 | *Nhe*I-Schnittstelle (verwendet für Klonierung) |
| 5930-6560 | pUC19-Backbone |

### Herstellung des Produktionsplasmides pSCIL043

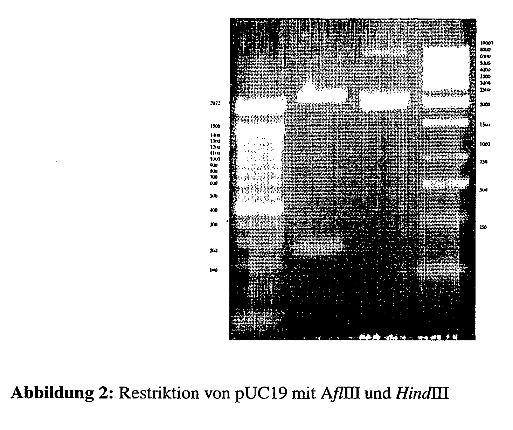

**1. Insertion eines Transkriptionsterminators in den Ausgangsvektor = pSCIL001**
   **1.1. Restriktion des Ausgangsvektors**
      - Ausgangsplasmid = Plasmid pUC19 bezogen von MBI-Fermentas
      - Restriktion des pUC19-Plasmides mit *Hin*dIII und *AfI*III (Abb. 2). Dadurch werden 359 bp aus dem Vektor deletiert.
   **1.2. Amplifikation des *t₀*-Terminators**
      - Amplifikation des *t₀*-Terminators mittels PCR durch folgende Primer (Abb. 3):
         1) *t₀*-OD-MCS-*Hin*dIII
            5'-AAAAAGCTT^{*Hind*III}GACTCCTGTTGATAGATCCAGTAA-3'
         2) *t₀*-UU-MCS*-Afl*III
            5'-AAAACATGT^{A*fl*III}ATTCTCACCAATAAAAAACGCC-3'
      - Restriktion des Terminatorfragmentes mit den Restriktionsendonukleasen *Hin*dIII (MBI) und *Afl*III (NEB) mit anschließender Reinigung des Fragmentes über den Minelute Kit (Qiagen)
      - Ligation des *t₀*-Terminators in pUC19^{*Hin*dIII/*Afl*III} Verifizierung des Einbaus durch Restriktionsanalyse und Sequenzierung = pSCIL001.
      **Austausch der Antibiotika-Resistenz in pSCIL001 = pSCIL002**
   **1.2. Amplifikation der Kanamycin-Kassette**
      - Amplifikation der Km-Kassette (990 bp) aus pACYC177 (NEB) mittels PCR durch folgende Primer (Abb. 4):
         1) Km-OD*-Apa*I
            5'- AAGGGCCC^{*Apa*I}GCCACGTTGTGTGTCTC-3
         2) Km-UU-*Nhe*I
            5'-AAAGCTAGC^{*Nhe*I}GATATCGCCGTCCCGTCAAGTG-3'
      - Zwischenklonierung des PCR-Produktes in pGEM-Teasy (Promega). Integritätsprüfung des Fragmentes erfolgte über Restriktionsanalyse und Sequenzierung der DNA.
   **2.2. Amplifikation von pSCIL001 ohne Ampicillin Resistenz Kassette**
      - Amplifikation des pSCIL001-Vektors (1462 bp) ohne Amp-Kassette (d.h. die verwendeten Primer flankierten die in pUC 19 vorhandene Amp-Kassette) mittels PCR erfolgte durch folgende Primer (Abb. 5):
         1) pUC2451-OD-*Nhe*I
            5'-AAAGCTAGC^{*Nhe*I}GGGAATAAGGGCGACAGGG-3'
         2) pUC1496-UU-*Apa*I
            5'-AAAGGGCCC^{*Apa*I}ACGTGAGTTTTCGTTCCACTG-3'
      - Die im Zwischenklonierungsvektor pGEM Teasy vorliegende Km-Kassette wurde über eine *Apa*I/*Nhe*I-Restriktion aus dem Vektor geschnitten und mit dem bereits vorher geschnittenen pSCIL001(ΔAmp)^{*Apa*I/*Nne*I}-Fragment (Abb. 5.) ligiert. Daraus entstand pSCIL002.
      - pSCIL002 wurde mittels Restriktionsanalyse untersucht. Dabei wurden im analytischen Maßstab vier verschiedene Restriktionen *(Apa*I/*Nhe*I*, Eco*RI*, Eco*RV und *Afl*III) durchgeführt. Der Vektor sollte bei allen Restriktionen linearisiert werden, nur die Behandlung mit *Apa*I und *Nhe*I sollte zum Herauslösen der einklonierten Km-Kassette führen (Abb. 6.).
**3. Insertion des sekundären Selektionsmarkers in pSCIL002 = pSCIL003**
   **3.1. Amplifikation der *proBA*-Determinante aus chromosomaler K12-DNA**
      - Amplifikation des *proBA*-Operons (2520 bp) mittels PCR erfolgte durch folgende Primer (Abb. 7). Als Template für die PCR wurde chromosomale K12-DNA verwendet. Diese DNA wurde mittels des DNeasy Tissue Kits (Qiagen) isoliert. Der *E. coli* K12-Stamm (DSMZ 903 7) wurde von DSMZ, Braunschweig, bezogen:
         1) *proAB-*OD*-Apa*I
            5'-AAAGGGCCC^{*Apa*I}GCAACCGACGACAGTCCTGC-3'
         2) *proAB-*UU*-Apa*I
            5'-AAAGGGCCC^{*Apa*I}CGGTGGACAAAGGTTAAAAC-3'
   **3.2. Klonierung der pro*BA*-Determinante in pSCIL002^{*Apa*I} und Funktionstest**
      - Das zwischenklonierte *proBA*-Fragment wurde durch eine *Apa*I-Restriktion aus dem pGEM Teasy-Vektor (Promega) herausgelöst und in den pSCIL002^{*Apa*I}-Vektor ligiert. Zur Überprüfung des richtigen Einbaus des sekundären Selektionsmarkers wurde der pSCIL003-Vektor mit *Eco*RV (NEB) geschnitten (Abb. 8).
      - Zur Überprüfung der Funktionalität des pSCIL003-Vektors, wurde der *E.coli*-Stamm JM83 mit den Vektoren pSCIL002 (ohne *proBA*) bzw. pSCIL003 (mit *proBA*) transformiert. Die entstandenen Transformanten wurde auf Mineralsalzmedium ausgestrichen und auf ihr Fähigkeit überprüft, dort zu wachsen (Abb. 9.). Eindeutig ist JM83 durch die Transformation mit pSCIL003 in der Lage auf Mineralsalzmedium zu wachsen. Ohne die *proBA-*Determinante auf einem Plasmid ist der Stamm dazu nicht in der Lage. Daher kann *proBA* als Selektionsmarker eingesetzt werden.
**4. Insertion des Repressors *lacI* = pSCIL004a**
   **4.1. Amplifikation des Repressors *lacI* aus chromosomaler K12-DNA**
      - Amplifikation des *lacI-*Gens inkl. des nativen Promotors (1160 bp) durch PCR erfolgte mittels folgender Primer (Abb. 10). Als Template für die PCR wurde chromosomale K12-DNA verwendet. Diese DNA wurde mittels des DNeasy Tissue Kits (Qiagen) isoliert. Der *E. coli* K12-Stamm (DSMZ 9037) wurde von der DSMZ Braunschweig bezogen:
         1) *lacI* OD *Nhe*I
            5'-AAAGCTAGC^{*Nhe*I}GACACCATCGAATGGCGC-3'
         2) *lacI* UU *Nhe*I
            5'-AAAGCTAGC^{*Nhe*I}TCACTGCCCGCTTTCC-3'
   **4.2. Einbau des Respressorgens in pSCIL003 = pSCIL004a**
      - Das Repressorgen wurde über die *Nhe*I-Schnittstelle in pSCIL003 eingefügt. Da es zwei Möglichkeiten zum Einbau des Repressorgens gab, wurden mittels Restriktionsanalyse die genaue Orientierung des Fragmentes bestimmt. Durch einen *Eco*RV-Verdau konnte die Lage des Gens in Expressionsrichtung des späteren Targetgens in der MCS gezeigt werden (Ergebnis nicht gezeigt).
**5. Insertion des *tac*-Promotors in pSCIL004a = pSCIL008**
   **5.1. Amplifikation des tac-Promotors und Klonierungsstrategie**
      - Die Amplifikation des *tac*-Promotors (67 bp) mittels PCR erfolgte durch folgende Primer. Als Template für die PCR wurde der Vektor pKK233-3 (Amersham, s. Anhang) verwendet.
         *1)* Pr*tac-Mun*I5*'*
            5'-AAACAATTG^{*Mun*I}TGTTGACAATTAATCATCGGCTC-3'
         3) Pr*tac*-*Eco*RI3'
            5'-AAAGAATTC^{*Eco*RI}TCTCCT^{RBS}TGTGAAATTGTTATCCGCTC-3'
      - Das PCR-Produkt wurde direkt mit den Restriktionsendonukleasen *Eco*RI und *Mun*I behandelt. Der 3'Primer Prtac *Eco*RI enthält neben der *Eco*RI-Site auch eine Ribosomenbindestelle. Durch die Ligation in den *Eco*RI geschnittenen pSCIL004a^{*Eco*RI} konnte der Promotor direkt upstream der *Eco*RI-Site in der MCS kloniert werden. Dabei wird durch die *Mun*I-Site am 5'Ende des Promotors die zweite *Eco*RI-Site zerstört. Der Einbau wurde durch Sequenzierung überprüft (Abb.11).
**6. Ligation des MIA-Gens**
   - Gensynthese des MIA-Gens durch die geneART GmbH, Regensburg
   - Das MIA-Fragment wurde mit den Restriktionsendonukleasen *Eco*RI und *Pst*I aus dem Syntheseplasmid (Abb. 12) geschnitten und in den genauso geschnittenen pSGIL008 Vektor ligiert. Der Einbau wurde durch Restriktion und Sequenzierung überprüft. Durch die Klonierung des MIA-Gens in das Plasmid pSCIL008 entstand der Expressionsvektor **pSCIL043**.
   - Der *E. coli*-Stamm JM83 wurde mit dem Expressionsplasmid pSCIL043 transformiert und auf Mineralsalzmedium durch mehrmaliges Ausstreichen auf festen Medien adaptiert. In einem Expressionsversuch (20 ml) wurde die Expressionsleistung des Vektors überprüft (Abb. 13).
   - Die Plasmidstabilität wurde durch die Testung der Zellen am Ende der Induktion auf selektivem und nicht selektivem (mit Kanamycin) LB-Agar untersucht. Dabei zeigte sich eine Stabilität von 100 % (Abb. 14).
**7. Konstruktionsschema**

### (Abb. 15)

### Beispiel 2: Anwendungsbeispiel für Fermentationsverfahren

Ein erfindungsgemäßer Expressionsvektor wird mit Methoden, die dem Stand der Technik entsprechen, in kompetente Zellen von *E. coli* JM83 sowie *E. coli* BL21 transformiert. Diese Zellen werden zuerst auf LB-Agar ausplattiert und bei 37°C inkubiert. Danach müssen sie an das Mineralsalzmedium adaptiert werden. Hierzu wird ein Klon von der LB-Agar-Platte auf eine Platte mit Mineralsalzmedium-Agar überimpft und bei 37°C inkubiert. Zur Verbesserung der Adaption an dieses Medium wird ein Klon von der Agar-Platte mit Mineralsalzmedium auf eine weitere Agar-Platte mit Mineralsalzmedium überimpft und bei 37°C inkubiert. Von dieser Platte werden 100 ml Mineralsalzmedium mit einem Klon angeimpft. Die Inkubation erfolgt bei 180 rpm mit 37°C über Nacht bis zu einer optischen Dichte von OD₆₀₀ = 3. Bei dieser OD werden von der Flüssigkultur Glycerol-Kulturen angelegt. Diese setzen sich zusammen aus 800 µl Zellsuspension und 200 µl Glycerol.

In diesem Anwendungsbeispiel werden zwei Fermentationen in 10 l Laborfermentern (B. Braun Biotech) beschrieben. Die erste wird mit *E. coli* JM83 prozessiert. Die zweite wird mit *E. coli* BL21, in dem der Selektionsmarker nicht aktiv ist, da BL21 nicht auxotroph ist, durchgeführt. Die Fermentationen werden als Fed-Batch-Prozesse durchgeführt. Das Batch-Volumen beträgt 6 1. Es werden 2 1 Substrat (Feed) dosiert, so dass sich ein Endvolumen von 8 1 ergibt.

Als Inokulat wird eine Glycerol-Kultur in 100 ml Mineralsalzmedium über Nacht bei 180 rpm mit 37°C inkubiert (1. Vorkultur). Vier Kolben mit 100 ml Mineralsalzmedium werden mit einem Teilvolumen der 1. Vorkultur angeimpft und bei 180 rpm mit 37°C inkubiert (2. Vorkultur). Bei einer definierten optischen Dichte, z. B. OD₆₀₀ = 3, wird die Zellsuspension zentrifugiert und das Zellpellet in 50 ml physiologischer Kochsalzlösung resuspendiert.

Die Batch-Phase startet mit der Inokulation und endet bei einer definierten optischen Dichte, z. B. OD₆₀₀ = 18, mit dem Start der Fed-Batch-Phase, d.h. mit dem Beginn der Substrat-Dosierung. Die Dosierung des Substrates (Feedflow) erfolgt nach einer exponentiellen Funktion der Form Feedflow = const * exp(µ₁ * t). Hierbei ist µ die spezifische Wachstumsrate, für die µ₁ = const < µₘₐₓ gilt. D.h., dass die jeweils momentane Substratmenge stets unter dem maximalen, momentanen Bedarf der Zellen liegt, wodurch eine submaximale Wachstumsrate erzielt wird. Es wird z. B. µ₁ = 0,35 h⁻¹ gewählt.

Bei einer definierten, optischen Dichte, z. B. OD₆₀₀ = 75, wird die Proteinexpression durch die Zugabe von IPTG, z. B. 1 mM, induziert. Zu diesem Zeitpunkt wird über die Dosierung des Substrates (Feedflow) eine andere spezifische Wachstumsrate µ₂ für die Zellen eingestellt, z. B. µ₂ = 0,1 h⁻¹. Der Prozess endet nach einer definierten Induktionsdauer, z.B. 4 h.

Der mit der steigenden Zelldichte sich stets erhöhende Sauerstoffbedarf wird über eine Kaskadenregelung für den Sauerstoffpartialdruck pO₂ konstant auf z. B. 20 % Sättigung gewährleistet. Hierzu erhöht sich bei steigendem Bedarf die Rührerdrehzahl. Bei Erreichen der maximalen Drehzahl erhöht sich die Begasungsrate mit Luft. Erlangt die Begasungsrate ihr Maximum, wird der Zuluft reiner Sauerstoff hinzudosiert.

Die Stickstoffversorgung erfolgt über die pH-Regelung, wobei Ammoniak als Base verwendet wird. Als Säure wird Phosphorsäure verwendet. Bei starker Schaumbildung wird automatisch Antischaummittel dosiert.

Zu verschiedenen Zeitpunkten der Fermentation werden Proben zur Bestimmung der Plasmidstabilität entnommen. Während es in BL21 sehr schnell zu einem totalen Verlust des Plasmides und damit der Proteinexpression kommt, kann in JM83 eine 100 %ige Plasmidstabilität während des gesamten Fermentationsprozesses nachgewiesen werden. Parallel zu diesen Proben wird aus verschiedenen Proben (im stündlichen Abstand im Verlauf der Fermentation) die Plasmide isoliert. Mittels verschiedener Endonukleasen wurde die Plasmidintegrität in JM83 bestimmt. Sie ist während des gesamten Fermentationsprozesses unbeeinflusst und die Restriktionsmuster zeigten die gleichen Banden wie im Ausgangsplasmid.

**Tabelle 1: Plasmidstabilitäten**

| Zeitpunkt | JM83 (pSCIL043) | BL21 (pSCEL043) |
|---|---|---|
| vor Induktion | 100 % | 35 % |
| nach Induktion | 100 % | 12 % |

## Patentansprüche

1. Expressionsvektor zur Verwendung in einer auxotrophen, prokaryontischen Wirtszelle, der die folgenden Bestandteile in funktioneller Verbindung umfasst:
a) eine regulatorische Sequenz,
b) eine für ein Protein/Peptid kodierende Sequenz,
c) ein erstes selektierbares Markergen, und
d) ein zweites selektierbares Markergen, wobei das Markergen ein vom auxotrophen Wirt nicht exprimiertes Protein kodiert, welches für die Biosynthese einer Aminosäure, in der die Wirtszelle auxotroph ist, notwendig ist,
bei dem die regulatorische Sequenz ein *tac*-Promotor mit einer Ribosomenbindestelle ist und der zweite selektierbare Marker *proBA* ist.

2. Expressionsvektor nach Anspruch 1, der weiterhin einen Terminator zur Termination der Transkription aufweist.

3. Expressionsvektor nach einem oder mehreren der vorhergehenden Ansprüche, der weiterhin ein Repressorgen aufweist.

4. Expressionsvektor nach einem oder mehreren der vorhergehenden Ansprüche, wobei das Repressorgen ein *lacI-*Gen ist.

5. Expressionsvektor nach einem oder mehreren der vorhergehenden Ansprüche, wobei die Ribosomenbindestelle die Sequenz AGGAGA aufweist.

6. Expressionsvektor nach einem oder mehreren der vorhergehenden Ansprüche, wobei das erste selektierbare Markergen ein Antibiotikaresistenzgen, vorzugsweise ein Kanamycinresistenzgen, ist.

7. Expressionsvektor nach einem oder mehreren der vorhergehenden Ansprüche, wobei die kodierende Sequenz für MIA, G-CSF, ProBMP, BMP, tPA, TNF, HGF, NGF, Proteasen wie Trypsin, Thrombin, Enterokinase, β-TGF, Interferone, Erythropoietin, Insulin, Faktor VII, Faktor VIII, Single Chain Antikörper, Affilin^{™} sowie Fusionen dieser Proteine, G-Protein-gekoppelte Rezeptoren sowie deren Domänen, sowie die Proformen dieser Proteine verwendet werden.

8. Expressionsvektor nach einem oder mehreren der vorhergehenden Ansprüche, wobei der Terminator *tₒ* aus dem Bakteriophagen Lambda ist.

9. Expressionsvektor nach einem oder mehreren der vorhergehenden Ansprüche, wobei der Expressionsvektor ein *high copy* Plasmid ist.

10. Expressionsvektor pSCIL008 nach einem oder mehreren der vorhergehenden Ansprüche, der folgende Bestandteile in funktioneller Verbindung enthält:
a) einen *tac*-Promotor mit einer Ribosomenbindestelle,
b) eine kodierende Sequenz für z.B. MIA, G-CSF, ProBMP, BMP, tPA, TNF, HGF, NGF, Proteasen wie Trypsin, Thrombin, Enterokinase, β-TGF, Interferone, Erythropoietin, Insulin, Faktor VII, Faktor VIII, Single Chain Antikörper, Affilin^{™} sowie Fusionen dieser Proteine, G-Protein-gekoppelte Rezeptoren sowie deren Domänen, sowie die Proformen dieser Proteine, GM-CSF, M-CSF, Interleukine, Interferone, Calcitonin, Caspase, VEGF, Factor III, Factor X, Factor Xa, Factor XII, Factor XIIa, GDF, IGF, Metalloproteasen, Antikörper, Antikörperfragmente, Immunotoxine.
c) ein Antibiotikaresistenzgen, vorzugsweise ein Kanamycinresistenzgen,
d) eine *proBA*-Sequenz,
e) wahlweise ein an den Operator des Promotors bindender Repressor, vorzugsweise das *lacl*-Repressorgen, und
f) wahlweise den *t₀*-Terminator aus Lambda zur Beendigung der Transkription eines Gens.

11. Expressionssystem, das die folgenden Bestandteile umfasst:
a) einen Expressionsvektor nach einem oder mehreren der Ansprüche 1-10, und
b) eine auxotrophe prokaryontische Wirtszelle.

12. Expressionssystem nach Anspruch 11, wobei die Wirtszelle eine auxotrophe *E*. *coli-*Zelle ist, die in der Aminosäure Prolin auxotroph ist.

13. Expressionssystem nach Anspruch 12, wobei die *E*. *coli*-Zelle aus den Stämmen JM I 06, JM108, JM109, JM83 und TB1 oder deren Derivaten ausgewählt ist.

14. Antibiotikafreies Fermentationsmedium, das ein Expressionssystem nach Anspruch 11-13 umfasst.

15. Fermentationsmedium nach Anspruch 14, das bei Vorliegen eines Repressorgens weiterhin einen Induktor umfasst.

16. Verfahren zur antibiotikafreien Expression von Peptiden/Proteinen, das die folgenden Schritte umfasst:
a) Transformieren von auxotrophen Wirtszellen mit einem Expressionsvektor nach Anspruch 1 - 10,
b) Selektion auf transformierte Wirtszellen, wobei die Selektion aufgrund der durch das zweite selektierbare Markergen exprimierten Aminosäure erfolgt;
c) Einbringen der transformierten Wirtszellen in ein antibiotikafreies Fermentationsmedium nach Anspruch 14 unter solchen Bedingungen, dass eine Fermentation stattfindet und das Protein/Peptid exprimiert wird; und
d) Isolieren und Aufreinigen des exprimierten Proteins/Peptids.

## Claims

1. An expression vector for use in an auxotrophic, prokaryotic host cell comprising the following components operably linked to each other:
a) a regulatory sequence,
b) a sequence coding for a protein/peptide,
c) a first selectable marker gene, and
d) a second selectable marker gene wherein the marker gene encodes a protein not expressed by the auxotrophic host which protein is necessary for the biosynthesis of an amino acid for which the host cell is auxotrophic,
wherein the regulatory sequence is a *tac* promoter containing a ribosomal binding site, and the second selectable marker is *proBA.*

2. The expression vector according to claim 1, further containing a terminator for termination of the transcription.

3. The expression vector according to one or more of the preceding claims further containing a repressor gene.

4. The expression vector according to one or more of the preceding claims wherein the repressor gene is a *lacI* gene.

5. The expression vector according to one or more of the preceding claims wherein the ribosomal binding site has the sequence AGGAGA.

6. The expression vector according to one or more of the preceding claims wherein the first selectable marker gene is an antibiotic resistance gene, preferably a kanamycin resistance gene.

7. The expression vector according to one or more of the preceding claims wherein the coding sequence for MIA, G-CSF, ProBMP, BMP, tPA, TNF, HGF, NGF, proteases such as trypsin, thrombin, enterokinase, β-TGF, interferons, erythropoietin, insulin, Factor VII, Factor VIII, single chain antibodies, Affilin^{™} as well as fusions of these proteins, G protein coupled receptors as well as the domains thereof and pro-forms of these proteins are used.

8. The expression vector according to one or more of the preceding claims wherein the terminator is *t₀* from bacteriophage Lambda.

9. The expression vector according to one or more of the preceding claims wherein the expression vector is a *high copy* plasmid.

10. The expression vector pSCIL008 according to one or more of the preceding claims containing the following components operably linked to each other:
a) a *tac* promoter having a ribosomal binding site,
b) a coding sequence for e.g. MIA, G-CSF, ProBMP, BMP, tPA, TNF, HGF, NGF, proteases such as trypsin, thrombin, enterokinase, β-TGF, interferons, erythropoietin, insulin, Factor VII, Factor VIII, single chain antibodies, Affilin^{™} as well as fusions of these proteins, G protein coupled receptors as well as the domains thereof, and the pro-forms of these proteins, GM-CSF, M-CSF, interleukins, interferons, calcitonin, caspase, VEGF, Factor III, Factor X, Factor Xa, Factor XII, Factor XIIa, GDF, IGF, metalloproteases, antibodies, antibody fragments or immunotoxins,
c) an antibiotic resistance gene, preferably a kanamycin resistance gene,
d) a *proBA* sequence,
e) optionally a repressor binding to the operator of the promoter, preferably the *lacI* repressor gene, and
f) optionally the *t₀* terminator from Lambda for termination of the transcription of a gene.

11. An expression system comprising the following components:
a) an expression vector according to one or more of the claims 1-10, and
b) an auxotrophic prokaryotic host cell.

12. The expression system according to claim 11 wherein the host cell is an auxotrophic *E. coli* cell which is auxotrophic for the amino acid proline.

13. The expression system according to claim 12 wherein the *E. coli* cell is selected from the strains JM106, JM108, JM109, JM83 and TB1 or the derivatives thereof.

14. An antibiotic-free fermentation medium comprising the expression system of claims 11-13.

15. The fermentation medium of claim 14, which further comprises an inducer, if a repressor gene is present.

16. A method for antibiotic-free expression of peptides/proteins comprising the following steps:
a) transforming auxotrophic host cells with an expression vector according to claim 1-10,
b) selecting for transformed host cells wherein the selection is performed on the basis of the amino acid expressed by the second selectable marker gene;
c) introducing the transformed host cells into an antibiotic-free fermentation medium according to claim 14 under conditions that fermentation occurs and the protein/peptide is expressed; and
d) isolating and purifying the expressed protein/peptide.

## Revendications

1. Vecteur d'expression destiné à l'utilisation dans une cellule hôte procaryote auxotrophe, qui comprend les composants suivants en liaison fonctionnelle :
a) une séquence régulatrice,
b) une séquence codant pour une protéine/un peptide,
c) un premier gène marqueur sélectionnable, et
d) un second gène marqueur sélectionnable, le gène marqueur codant une protéine non exprimée par l'hôte auxotrophe, qui est nécessaire pour la biosynthèse d'un acide aminé pour lequel la cellule hôte est auxotrophe,
dans lequel la séquence régulatrice est un promoteur *tac* avec un site de fixation des ribosomes et le second marqueur sélectionnable est *proBA.*

2. Vecteur d'expression selon la revendication 1, qui comporte en outre un terminateur pour la terminaison de la transcription.

3. Vecteur d'expression selon une ou plusieurs des revendications précédentes, qui comporte en outre un gène répresseur.

4. Vecteur d'expression selon une ou plusieurs des revendications précédentes, dans lequel le gène répresseur est un gène *lacI.*

5. Vecteur d'expression selon une ou plusieurs des revendications précédentes, dans lequel le site de fixation du ribosome est la séquence AGGAGA.

6. Vecteur d'expression selon une ou plusieurs des revendications précédentes, dans lequel le premier gène marqueur sélectionnable est un gène de résistance à un antibiotique, de préférence un gène de résistance à la kanamycine.

7. Vecteur d'expression selon une ou plusieurs des revendications précédentes, dans lequel on utilise la séquence codante pour MIA, G-CSF, ProBMP, BMP, tPA, TNF, HGF, NGF, des protéases telles que la trypsine, la thrombine, l'entérokinase, le TGF-β, des interférons, l'érythropoïétine, l'insuline, le facteur VII, le facteur VIII, des anticorps monocaténaires, l'Affilin^{™} ainsi que des fusions de ces protéines, des récepteurs couplés à la protéine G ainsi que leurs domaines, ainsi que les proformes de ces protéines.

8. Vecteur d'expression selon une ou plusieurs des revendications précédentes, dans lequel le terminateur *t*₀ provient du bactériophage Lambda.

9. Vecteur d'expression selon une ou plusieurs des revendications précédentes, dans lequel le vecteur d'expression est un plasmide *high copy.*

10. Vecteur d'expression pSCIL008 selon une ou plusieurs des revendications précédentes, qui contient les composants suivants en liaison fonctionnelle :
a) un promoteur *tac* comportant un site de fixation des ribosomes,
b) une séquence codante par exemple pour MIA, G-CSF, ProBMP, BMP, tPA, TNF, HGF, NGF, des protéases telles que la trypsine, la thrombine, l'entérokinase, le TGF-β, des interférons, l'érythropoïétine, l'insuline, le facteur VII, le facteur VIII, des anticorps monocaténaires, l'Affilin^{™} ainsi que des fusions de ces protéines, des récepteurs couplés à la protéine G ainsi que leurs domaines, ainsi que les proformes de ces protéines, GM-CSF, M-CSF, des interleukines, des interférons, la calcitonine, la caspase, le VEGF, le facteur III, le facteur X, le facteur Xa, le facteur XII, le facteur XIIa, le GDF, l'IGF, des métalloprotéases, des anticorps, des fragments d'anticorps, des immunotoxines,
c) un gène de résistance à un antibiotique, de préférence un gène de résistance à la kanamycine,
d) une séquence *proBA,*
e) éventuellement, un répresseur se liant à l'opérateur du promoteur, de préférence le gène répresseur *lacI,* et
f) éventuellement, le terminateur *tₒ* provenant de Lambda pour la terminaison de la transcription d'un gène.

11. Système d'expression qui comprend les composants suivants :
a) un vecteur d'expression selon une ou plusieurs des revendications 1 à 10, et
b) une cellule hôte procaryote auxotrophe.

12. Système d'expression selon la revendication 11, dans lequel la cellule hôte est une cellule d'*E. coli* auxotrophe, qui est auxotrophe pour l'acide aminé proline.

13. Système d'expression selon la revendication 12, dans lequel la cellule d'*E. coli* est choisie parmi les souches JM106, JM108, JM109, JM83 et TB1 ou leurs dérivés.

14. Milieu de fermentation sans antibiotique, qui comprend un système d'expression selon l'une quelconque des revendications 11 à 13.

15. Milieu de fermentation selon la revendication 14, qui en présence d'un gène répresseur comprend en outre un inducteur.

16. Procédé pour l'expression sans antibiotique de peptides/protéines, qui comprend les étapes suivantes :
a) transformation de cellules hôtes auxotrophes par un vecteur d'expression selon l'une quelconque des revendications 1 à 10,
b) sélection des cellules transformées, la sélection s'effectuant sur la base de l'acide aminé exprimé par le second gène marqueur sélectionnable ;
c) introduction des cellules hôtes transformées dans un milieu de fermentation sans antibiotique selon la revendication 14, dans des conditions telles qu'une fermentation a lieu et la protéine/le peptide est exprimé(e) ; et
d) isolement et purification de la protéine/du peptide exprimé(e).
